**Europäisches Patentamt**

**European Patent Office**

**Office européen des brevets**

(19)

(11) Veröffentlichungsnummer: **0 281 504 B1**

# (12) EUROPÄISCHE PATENTSCHRIFT

(45) Veröffentlichungstag der Patentschrift:
06.11.91 Patentblatt 91/45

(51) Int. Cl.⁵: **C21C 7/10**

(21) Anmeldenummer: 88730031.7

(22) Anmeldetag: 09.02.88

(54) **Verfahren und Vorrichtung zur Entgasungsbehandlung einer Stahlschmelze in einer Vakuumanlage.**

(30) Priorität: 28.02.87 DE 3706742

(43) Veröffentlichungstag der Anmeldung:
07.09.88 Patentblatt 88/36

(45) Bekanntmachung des Hinweises auf die
Patenterteilung:
06.11.91 Patentblatt 91/45

(84) Benannte Vertragsstaaten:
DE FR IT

(56) Entgegenhaltungen:
DE-A- 2 462 540
DE-A- 3 428 732
US-A- 3 520 657
US-A- 3 748 122

(73) Patentinhaber: Stahlwerke Peine-Salzgitter
AG
Gerhardstrasse 10
W-3150 Peine (DE)

(72) Erfinder: Nolle, Dieter, Dr.
Fontaneweg 40
W-3340 Wolfenbüttel (DE)
Erfinder: Eulenburg, Ulrich, Dr.
Humboldt-Allee 25
W-3320 Salzgitter 1 (DE)
Erfinder: Jahns, Armin
Saldersche Strasse 40
W-3320 Salzgitter 1 (DE)
Erfinder: Gramatzki, Alfred
Neissestrasse 28
W-3320 Salzgitter 1 (DE)
Erfinder: Evers, Wilhelm
Waldenburger Strasse 13
W-3340 Wolfenbüttel (DE)

(74) Vertreter: Kaiser, Henning
Preussag AG, Patente und Lizenzen, Postfach
15 12 27, Kurfürstendamm 32
W-1000 Berlin 15 (DE)

**Beschreibung**

Die Erfindung betrifft ein Verfahren und eine Vorrichtung zur Entgasungsbehandlung einer Stahlschmelze, wobei die Behandlungsdauer durch gemessene Prozeßdaten gesteuert wird.

Die Behandlungsdauer soll einerseits die Einstellung der Gehalte von Schmelzenbestandteilen, die durch die Entgasungsbehandlung beeinflußt werden, gewährleisten, andererseits jedoch nicht länger als notwendig sein, da dies zu erhöhten Gewinnungskosten des Stahls führt.

Es ist bekannt, daß durch eine Absenkung des Drucks über der Stahlschmelze gasbildende Stoffe z.B. Kohlenstoff und Wasserstoff entfernt werden können. Zur Entfernung des Kohlenstoffs ist die Anwesenheit von Sauerstoff in der Schmelze erforderlich, um den Kohlenstoff in der Verbindung Kohlenmonoxid entfernen zu können. Der benötigte Sauerstoff kann durch Einblasen oder Aufblasen von Sauerstoff oder durch die Zugabe von sauerstoffhaltigem Material (z.B. Eisensinter) während der Entgasungsbehandlung oder bereits in dem vorgeschalteten Verfahrensschritt der Blasstahlbehandlung bereitgestellt werden. Die notwendigen Sauerstoffgehalte richten sich nach der Menge des zu entfernenden Kohlenstoffs gemäß dem bekannten "Vacher-Hamilton"-Diagramm ("Stahlerzeugung" von H. Burghardt und G. Neuhof, VEB Deutscher Verlag für Grundstoffindustrie, Leipzig, 1. Auflage, Seite 425 ff.) Die Entgasungsbehandlung zur Entkohlung der Schmelze auf Gehalte unter 0,03 Gew.% bis unter 0,01 Gew.% für korrosionsfeste Stähle, super ferritische Stähle, Transformatorstähle usw. soll mit dem geringstmöglichen Aufwand an Betriebsmitteln erfolgen. Während der Entgasungsbehandlung sind produktbezogene Kosten u.a. durch den Betrieb der Vakuumanlage (Energiekosten) und den Verschleiß der Pfannenausmauerung gegeben. Für die wirtschaftliche Durchführung der Entgasungsbehandlung ist es notwendig, die Behandlung zu beenden, sobald die Endanalyse der Schmelze erreicht ist. Bei den bekannten Verfahren zur Entgasungsbehandlung richtet sich die Behandlungsdauer entweder in empirischer Weise nach den Angaben des Herstellers der Anlage oder nach Erfahrungswerten der Betriebspraxis oder nach steuerungstechnisch aufwendigen Verfahren der Analyse des Abgases der Entgasungsanlage, bei denen aus dem Kohlenstoffgehalt des Abgases in Form von Kohlenmonoxid und Kohlendioxid und der Abgasmenge der zeitliche Verlauf des Kohlenstoffgehaltes in der Schmelze bestimmt wird.

Grundsätzlich ist eine Steuerung des Entgasungsverfahrens aufgrund von Prozeßdaten der empirischen Fahrweise vorzuziehen.

Es ist vorgeschlagen worden, ("Stahl und Eisen", 92 (1972), Heft 25, Seite 1278/1283) das Abgas der Entgasungsanlage massenspektrometrisch zu analysieren. Die DE-PS 2839315 beschreibt ein massenspektrometrisches Verfahren zur Steuerung der Stahlerzeugung. Dort wird neben der Gasanalyse auch eine Bestimmung der Abgasmenge als verfahrensnotwendig bezeichnet. Die Bestimmung der Abgasmenge ist in einer Entgasungsanlage für Stahlschmelzen problematisch. Nach DE-PS 2839315 wird dazu die Einführung eines Bezugsgases vorgesehen, das in definierter Menge eingebracht wird und aus dessen Verdünnung im Abgas der Entgasungsanlage die Abgasmenge berechnet werden kann.

Nach DE-OS 3428732 wird bei einem Verfahren zur Erzeugung von Stählen mit niedrigem Kohlenstoffgehalt im Vakuum durch Sauerstoffeinblasen der Kohlenstoffendpunkt und die Einblastemperatur geregelt, indem die Schmelze mit Argon gekühlt wird und unter anderem die Zusammensetzung, Temperatur und Menge der Rauchgase bestimmt wird. Dabei soll die Argonintensität geregelt werden.

In den Rauchgasen soll der Gehalt an Kohlenmonoxid, Kohlendioxid und Sauerstoff gemessen werden. Über die Gesamtsauerstoffmenge und die Menge des Kohlendioxids soll das Sauerstoffeinbasen gesteuert werden.

Über die Temperatur und über die Kohlenmonoxid- sowie Kohlendioxidwerte im Rauchgas soll die Lanzenstellung geregelt werden.

Nach DE-PS 3925425 wird die Vakuumentgasung unter Einleiten von Sauerstoff zur Verminderung des Spritzens durch voreingestellte Werte der Fließgeschwindigkeit des Sauerstoffs und des Drucks im Entgasungsgefäß gesteuert.

Der mit dem Abgas entfernte Kohlenstoff soll bestimmt werden und daraus soll der Kohlenstoffgehalt der Schmelze berechnet werden. Der berechnete Kohlenstoffgehalt soll durch Probennahmen und Analysen der Proben überprüft werden.

Bei beiden Verfahren ist die Messung von Bestandteilen des Abgases der Entgasungsanlage sowie dessen Temperatur und Menge notwendig. Dies ist mit erheblichem apparativen Aufwand verbunden.

Der Einsatz eines Massenspektrometers im Stahlwerk und die bisher als notwendig erachtete Bestimmung der Abgasmenge sind mit relativ hohem Aufwand verbunden. Die Geräte sind teuer, benötigen einen angeschlossenen Rechner, relativ staubfreie Räume, sowie zusätzlich zur Stahlwerksmannschaft höher qualifiziertes Bedienungspersonal.

Aus US-PS 3520657 ist ein Verfahren und eine Vorrichtung zur Analyse des Abgases eines Raffinationsprozesses insbesondere für Stahl und hier speziell für die Entfernung von Kohlenstoff aus dem geschmolzenen Stahl bekannt. Bei diesem Verfahren wird ausgehend vom Kohlenstoffgehalt und dem Gewicht der Stahlschmelze sowie aus dem im Abgas entfernten Kohlenstoff in Form von Kohlenmonoxid die Restmenge Kohlenstoff in der Schmelze bilanziert. Um den Gewichtsanteil von Kohlenmonoxid im Abgas bestimmen zu können, muß dem Abgas ein Führungsgas (Tracer) in bekannter Menge zugegeben werden.

Der Erfindung liegt die Aufgabe zugrunde, ein selbtgängiges Verfahren ohne Sauerstoffeinblasen während der Entgasungsbehandlung einer Stahlschmelze anzugeben, die gasbildende Stoffe in ausreichender Menge enthält, wobei durch Messungen von einzelnen Bestandteilen eines verdichteten Gasstromes, der aus dem Abgas- oder Hauptvakuumstrom abgezweigt wird, die für einen gewünschten Restgehalt an gasbildender Substanz, insbesondere an Kohlenstoff, notwendige Dauer der Entgasungsbehandlung gesteuert wird. Aufgabe der Erfindung ist auch die Schaffung einer geeigneten Vorrichtungsanordnung zur Durchführung des Verfahrens.

Die Lösung dieser Aufgabe ist in den Ansprüchen 1 und 5 angegeben. Die Unteransprüche 2 und 3 beziehen sich auf bevorzugte weitere Ausgestaltungen, insbesondere des Meßverfahrens. Die Ansprüche 4 und 6 beziehen sich auf ein bevorzugtes Ausführungsbeispiel.

Nach der Erfindung wird demgemäß die Entgasungsbehandlung entsprechend dem relativen Volumenanteil wenigstens eines mit dem Abgas aus dem flüssigen Stahl entweichenden Gases gesteuert, das den aus der Stahlschmelze zu entfernenden Stoff enthält. Der relative Volumenanteil dieses Gases ist bezeichnend für den noch in der Schmelze vorhandenen Restgehalt des zu entfernenden Stoffes. Es ist damit möglich, die Vakuumbehandlung zu beenden, wenn in einem aus dem Abgasstrom abgezweigten Gasstrom der relative Volumenanteil des zu untersuchenden Gases einen vorbestimmten Minimalwert erreicht oder unterschreitet, da dieser einem erwünschten Sollwert des Stoffes in der Schmelze entspricht.

Eine Bestimmung der gesamten Abgasmenge ist entgegen der aus dem Stand der Technik bekannten Auffassung nicht notwendig. Die Ermittlung des relativen Volumenanteils in einem aus dem Abgas abgezweigten und vedichteten Gasstrom ermöglicht die Verwendung einfacher Meßgeräte. Es kann sowohl der verdichtete Gasstrom als auch ein Teil davon auf seine Zusammensetzung analysiert werden. Die Analyse kann auf die Bestimmung der Volumenanteile einzelner Bestandteile in einer speziellen Anwendung auf die Bestandteile Kohlenmonoxid, Kohlendioxid und Wasserstoff beschränkt werden.

Die Entgasungsbehandlung wird beendet, wenn ein oder mehrere Bestandteile des aus dem Abgas abgezweigten Gasstromes oder eines hiervon abgezweigten Teilstromes Minimalwerte (Zielwerte) erreicht haben, die Gehalten an gasbildenden Bestandteilen der Schmelze entsprechen, wie sie von der Zielanalyse der produzierten Stahlsorte vorgegeben sind.

Die mit der Erfindung erzielten Vorteile bestehen insbesondere darin, daß mit geringem Sach- und Personalaufwand einerseits Produktionskosten (Energiekosten und Feuerfestkosten) eingespart, sowie andererseits die Auslastung der Entgasungsanlage verbessert werden kann.

Ein Ausführungsbeispiel der Erfindung ist in den Abbildungen schematisch dargestellt.

Es zeigen :

Fig. 1 eine Entgasungsanlage mit Entnahmeeinrichtung zur Analyse des Abgases,

Fig. 2 eine spezielle Ausführung zur Verdichtung und Analyse des aus dem Abgas entnommenen Meßgases.

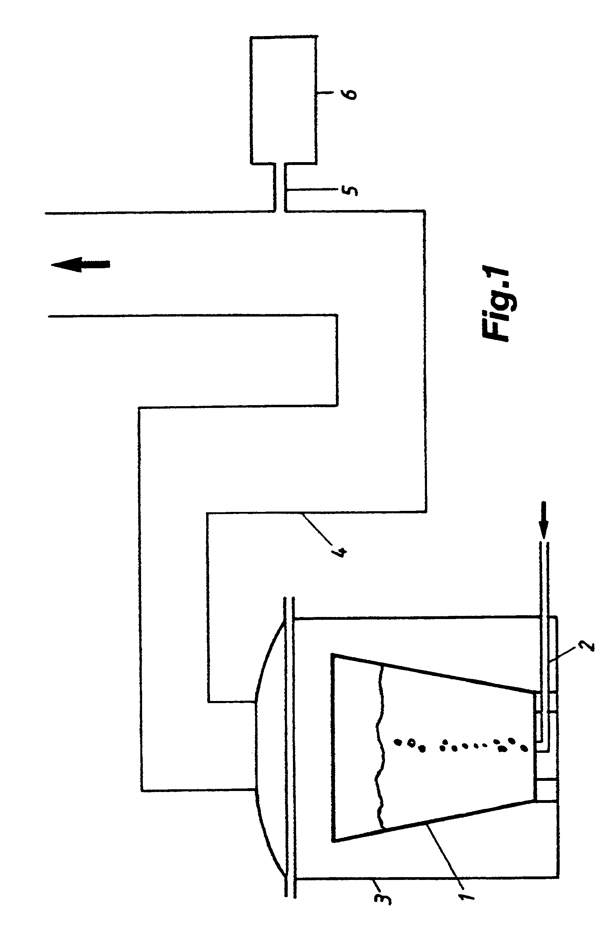

In Fig. 1 ist eine mit flüssigem Stahl gefüllte Pfanne (1) dargestellt, in die über eine Zuleitung (2) Inertgas (Argon) eingeleitet wird. Die Pfanne steht in einem Vakuumbehälter (3) mit beweglichem Deckel, an den die zu den nicht dargestellten Vakuumpumpen führende Abgasleitung (4) angeschlossen ist.

Aus der Abgasleitung (4) wird an einer Stelle, an der eine homogene Zusammensetzung zu erwarten ist, über einen Abzweig (5) mit einer Verdichtungs- und Analyseeinheit (6) kontinuierlich Meßgas entnommen und analysiert.

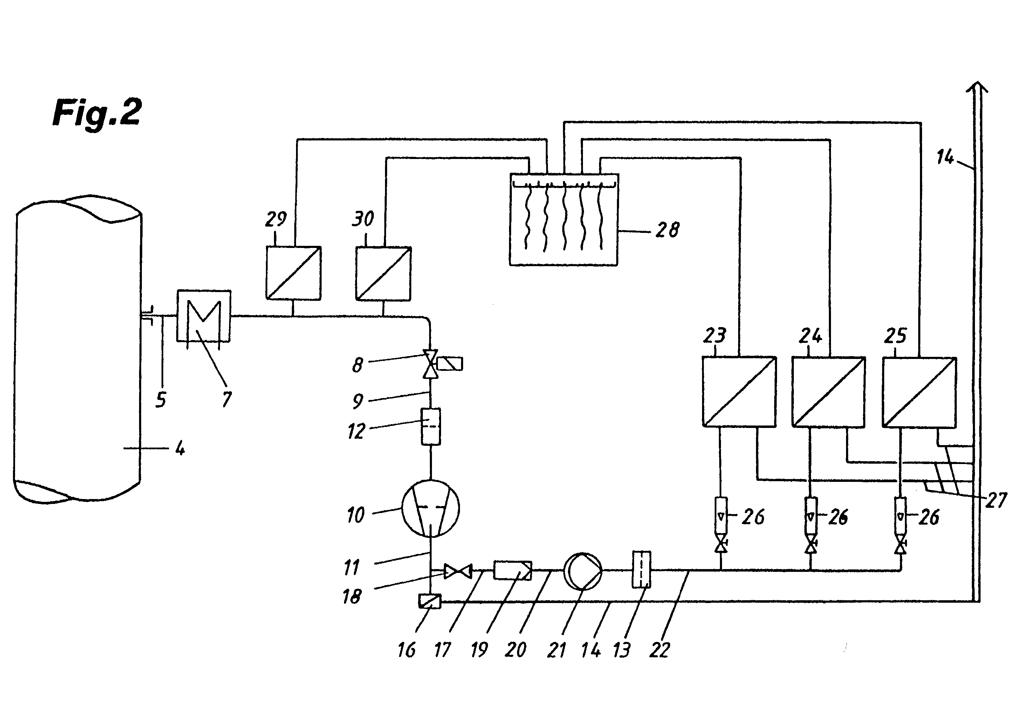

In Fig. 2 ist eine spezielle Ausführung der Einrichtung zur Entnahme, Verdichtung und Analyse von Meßgas aus der Abgasleitung (4) dargestellt. Über eine Leitung (5) und eine Kühleinrichtung (7) sowie ein Absperrventil (8) und eine Leitung (9) wird mit der Vakuumpumpe (10), der eine Filtereinrichtung (12) vorgeschaltet ist, ein Gasstrom aus dem Abgasstrom entnommen. Die Vakuumpumpe (10) für den verdichtenden Gasstrom hat einen Ölabscheider (nicht dargestellt). Der Gasstrom wird von der Vakuumpumpe (10) über eine Leitung (11) und eine Rückschlagklappe (16) in eine Abgasleitung (14) gefördert. Von der Leitung (11) zweigt die Leitung (17) mit dem Ventil (18) zu dem Filter (19) ab. Der Filter (19) ist über die Leitung (20) mit einer Meßgasförderpumpe (21) verbunden. Die Meßgasförderpumpe (21) fördert über die Leitung und einen Feinfilter (13) einen Teilstrom in die Infrarot-Analysatoren (23, 24) und den Wärmeleit-Analysator (25). Die Analysatoren enthalten geeignete, nicht dargestellte Korrektureinrichtungen. Vor den Analysatoren (23, 24, 25) sind Durchflußmeß- und Regelgeräte (26) angeordnet. Die Meßgase werden von den Analysegeräten über Leitungen (27) in die Abgasleitung (14) geleitet. Das Ergebnis der Analyse wird in einem Anzeigegerät (28) dargestellt ; ebenso der Druck in Leitung (5) über Druckmeßgeräte (29, 30).

In einer speziellen Ausführung des erfindungsgemäßen Verfahrens wird die Entkohlung von 160 t-Schmelzen auf Kohlenstoffgehalte unter 0,01 Gew.% durchgeführt. Dabei wird das Verfahren selbstgängig geführt, d.h., der für die Entkohlung notwendige Sauerstoffgehalt wird bereits in dem vorgeschalteten Verfahrensschritt, der Frischbehandlung, eingestellt. Mit Hilfe von angeschlossenen Dampfstrahlpumpen oder nicht dargestellte, sonstigen Vakuumpumpen wird der Atmosphärendruck in der Entgasungsanlage, also in dem Vakuumbehälter (3), auf weniger als 0,7 mbar abgesenkt. Gleichzeitig wird ein Inertgas (Argon) zur Spülung der Schmelze in die Pfanne (1) eingeblasen (Fig. 1). Das aus der Abgasleitung (4) mittels der Vakuumpumpe (10) entnommene Meßgas wird auf mehr als 1 bar verdichtet und in den Analysengeräten (23, 24, 25) der Gehalt an Kohlenmonoxid, Kohlendioxid, Wasserstoff in Vol.% bestimmt (Fig. 2). Die Entgasungsbehandlung wird beendet, wenn der Gehalt an Kohlenmonoxid im Meßgas unter 0,1 Vol.% gesunken ist. Der Kohlenstoffgehalt in der Schmelze beträgt dann weniger als 0,01 Gew.%. Betriebsüblich wurden vor Einsatz des erfindungsgemäßen Verfahrens Behandlungszeiten von 20 Minuten benötigt. Durch die Anwendung des erfindungsgemäßen Verfahrens konnten die Behandlungszeiten erfahrungsgemäß um 10 bis 20% vermindert werden. In einzelnen Fällen konnte die Behandlungsdauer auf 50% vermidert werden. Höchst ausnahmsweise mußte die Behandlungsdauer auch mal vergrößert werden ; die nach dem üblichen Verfahren behandelte Schmelze hätte in diesem Fall nicht die Zielanalyse erreicht und wäre mit entsprechendem Verlust als Schrott einzustufen gewesen.

## Patentansprüche

1. Verfahren zur Entgasungsbehandlung einer Stahlschmelze nach dem Frischen in einer Vakuumanlage, wobei aus der Schmelze gasförmige Bestandteile durch Verminderung des Atmosphärendruckes auf unter 1 mbar über der Schmelze entfernt werden und so ein Abgasstrom erzeugt wird, dessen Bestandteile, wenigstens Kohlenmonoxid, gemessen werden, dadurch gekennzeichnet, daß ausschließlich aus dem Abgasstrom der Vakuumanlage an einem Ort, an dem das Abgas homogen vermischt ist, ein Gasstrom abgezweigt und verdichtet wird und die Vakuumbehandlung beendet wird, wenn einer der gemessenen Bestandteile des abgezweigten und verdichteten Gasstromes einen vorbestimmten Minimalwert unterschreitet.

2. Verfahren gemäß Anspruch 1, dadurch gekennzeichnet, daß aus dem abgezweigten und verdichteten Gasstrom ein Teilstrom abgezweigt und dieser auf seine Zusammensetzung analysiert wird.

3. Verfahren gemäß Anspruch 1 oder 2, dadurch gekennzeichnet, daß die Volumenanteile von Kohlenmonoxid und/oder Kohlenoxid und/oder Wasserstoff bestimmt werden.

4. Verfahren gemäß einem oder mehreren der Ansprüche 1 bis 3, dadurch gekennzeichnet, daß bei der Entfernung von Kohlenstoff aus einer Schmelze von ca. 160 t Gewicht die Entgasung beendet wird, wenn bei einem Atmosphärendruck von weniger als 0,7 mbar über der Schmelze der Abgasanteil von Kohlenmonoxid 0.1 Vol.% beträgt.

5. Vorrichtung zur Durchführung eines Verfahrens nach einem oder mehreren der Ansprüche 1 bis 4 mit einer Vakuumbehandlungsanlage für Stahlschmelzen (1, 2, 3, 4) und einem Analysator für Bestandteile des Abgases, dadurch gekennzeichnet, daß von der Abgasleitung (4) eine ausschließlich Abgas enthaltende Abzweigleitung (5, 9) zu einer auf mehr als 1 bar verdichtenden Vakuumpumpe (10) führt, aus deren druckseitiger Leitung (11) eine weitere Abzweigleitung (17) zu einer einen Teilstrom fördernden Meßgasförderpumpe (21) führt und deren druckseitige Leitung (22) mit Analysatoren (23, 24, 25) für Kohlenmonoxid, Kohlendioxid und Wasserstoff verbunden ist.

6. Vorrichtung gemäß Anspruch 5, gekennzeichnet durch ein von den Meßergebnissen eines Wärmeleitanalysators (25) gespeistes Korrekturgerät für einen Infrarotanalysator (23, 24).

## Claims

1. Method for the degassing treatment of a molten steel mass subsequent to the refining process in a vacuum installation, gaseous constituent ingredients being removed from the molten mass by reducing the atmospheric pressure to below 1 mbar above the molten mass, and a stream of waste gas being produced thereby, the constituent ingredients of which stream, at least carbon monoxide, being measured, characterised in that a gas stream is separated from the stream of waste gas in the vacuum plant and compressed at a location, where the waste gas is homogeneously mixed, and the vacuum treatment is terminated, when one of the measured constituent ingredients of the separated and compressed gas stream falls below a predetermined minimum value.

2. Method according to claim 1, characterised in that a partial stream is separated from the separated and compressed gas stream, and its composition is analyzed.

3. Method according to claim 1 or 2, characterised in that the parts by volume of carbon monoxide and/or carbon dioxide and/or hydrogen are determined.

4. Method according to one or more of claim 1 to 3, characterised in that, upon the removal of carbon from a molten mass weighing approx. 160 t, the degassing process is terminated when the carbon monoxide content in the waste gas is 0.1% by vol. at an atmospheric pressure of less than 0.7 mbar above the molten mass.

5. Device for implementing a method according to one or more of claims 1 to 4, having a vacuum treatment installation for treating molten steel masses (1, 2, 3, 4) and an analyzer for analyzing constituent ingredients of the waste gas, characterised in that a branch conduit (5, 9), which exclusively contains waste gas, extends from the waste gas conduit (4) to a vacuum pump (10), which compresses to more than 1 bar, an additional branch conduit (17) extending from the conduit (11) at the pressure end of the vacuum pump to a measuring gas feed pump (21), which conveys a partial stream, and the conduit (22) at the pressure end of said pump being connected to analyzers (23, 24, 25) for analyzing carbon monoxide, carbon dioxide and hydrogen.

6. Device according to claim 5, characterised by a correcting means for an infra-red analyzer (23, 24), said means being supplied with the measurement results of a heat-conduction analyzer (25).

## Revendications

1. Procédé pour le dégazage d'acier fondu après l'affinage dans une installation à vide, selon lequel des éléments gazeux sont extraits de la masse fondue grâce une réduction de la pression atmosphérique, qui devient inférieure à 1 mbar, au-dessus de la masse fondue, ce qui produit un courant de gaz d'échappement dont les éléments, au moins l'oxyde de carbone, sont mesurés, caractérisé en ce que, à partir du courant de gaz d'échappement de l'installation sous vide, à un endroit où les gaz d'échappement sont mélangés de façon homogène, un courant de gaz est dérivé et comprimé, et le traitement sous vide est terminé lorsque l'un des éléments mesurés du courant de gaz dérivé et comprimé est inférieur à une valeur minimale prédéterminée.

2. Procédé selon la revendication 1, caractérisé en ce qu'un courant partiel est dérivé à partir du courant de gaz dérivé et comprimé, et la composition de ce courant partiel est analysée.

3. Procédé selon la revendication 1 ou 2, caractérisé en ce que les parts de volume d'oxyde de carbone et/ou de dioxyde de carbone et/ou d'hydrogène sont définies.

4. Procédé selon l'une au moins des revendications 1 à 3, caractérisé en ce que, lorsque le carbone est éliminé d'une masse fondue pesant environ 160 t, le dégazage est terminé quand, en présence d'une pression atmosphérique inférieure à 0,7 mbar au-dessus de la masse fondue, la part d'oxyde de carbone dans les gaz d'échappement est de 0,1% de volume.

5. Dispositif pour mettre en oeuvre un procédé selon l'une au moins des revendications 1 à 4, comportant une installation de traitement sous vide (1, 2, 3, 4) pour des aciers fondus et un analyseur pour les éléments du gaz d'échappement, caractérisé en ce que, à partir de la conduite de gaz d'échappement (4), une conduite de dérivation (5, 9) contenant exclusivement des gaz d'échappement, mène à une pompe à vide (10) effectuant une compression supérieure à 1 bar, dont la conduite côté refoulement (11) débouche sur une seconde conduite de dérivation (17) menant à une pompe d'alimentation de gaz à mesurer (21) alimentant un courant partiel, et dont la conduite côté impression (22) est reliée à des analyseurs (23, 24, 25) pour l'oxyde de carbone, le dioxyde de carbone et l'hydrogène.

6. Dispositif selon la revendication 5, caractérisé par un appareil de correction pour un analyseur d'infrarouge (23, 24), alimenté par les résultats de mesure d'un analyseur de conductibilité thermique (25).

**Fig.1**

EP 0 281 504 B1

Fig.2

EP 0 281 504 B1